Europäisches Patentamt
European Patent Office
Office européen des brevets

(19)

(11) Publication number: **0 224 951 A1**

(12) **EUROPEAN PATENT APPLICATION**

(21) Application number: **86202013.8**

(22) Date of filing: **17.11.86**

(51) Int. Cl.[4]: **G03C 7/26**, G03C 5/54, //C07D211/58,C08F220/38

(30) Priority: **26.11.85 EP 85201962**

(43) Date of publication of application: **10.06.87 Bulletin 87/24**

(84) Designated Contracting States: **BE DE FR GB**

(71) Applicant: **AGFA-GEVAERT naamloze vennootschap**
**Septestraat 27**
**B-2510 Mortsel(BE)**

(72) Inventor: **Van de Sande, Christian Charles**
**Tuinlaan 95**
**B-9180 Belsele(BE)**
Inventor: **Vanmaele, Luc Jerome**
**Hubert Frère Orbanlaan 231**
**B-9000 Gent(BE)**
Inventor: **Monballu, Marcel Jacob**
**Neuris 9**
**B-2510 Mortsel(BE)**
Inventor: **Janssens, Wilhelmus**
**De Egdstraat 11**
**B-3240 Aarschot(BE)**

(54) **Dye image-receiving element comprising anti-fading agents.**

(57) Dye image-receiving element, which in an image-receiving layer or in a hydrophilic colloid layer comprises as an anti-fading agent a 2,2,6,6-tetramethyl-substituted piperidine or piperidinium salt of the formulae I and II:

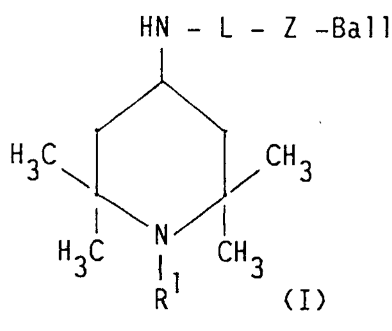
(I)

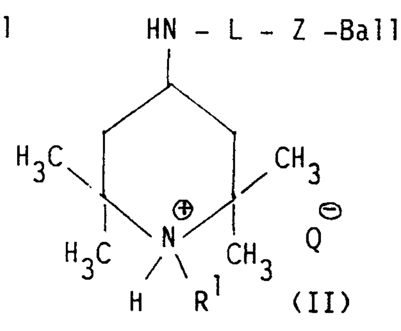
(II)

wherein:
wherein L is -CO-, -CO-CO-, or $-SO_2-$; Z is a single bond, -O-or $-N(R^2)-$, $R^2$ being H, alkyl, aryl, aralkyl, or a heterocyclic group; Ball is a ballasting group; $R^1$ is H, alkyl, or aralkyl; $Q^-$ is a mono-or polyatomic anion; and/or a polymer comprising recurring structural units, at least some of which are derived from the above anti-fading agents. The invention also provides a dye image-carrying element comprising (a) layer(s) in which a magenta, cyan, and/or yellow dye has (have) been formed by diffusion transfer and comprising an anti-fading agent as defined above.

EP 0 224 951 A1

# DYE IMAGE-RECEIVING ELEMENT COMPRISING ANTI-FADING AGENTS

The present invention relates to a dye image-receiving element, which comprises a 2,2,6,6-tetramethyl-piperidine compound in an image-receiving layer or in a hydrophilic colloid layer in water-permeable relationship therewith.

The expression "dye image-receiving element" denotes an element that comprises a layer, in which a dye image can be formed by a diffusion transfer technique. The expression "dye image-carrying element" as used hereafter denotes any such image-receiving element, in which a said dye image has already been formed.

It is known to form a dye image in an image-receiving layer by a method utilising a diffusion transfer imaging method e.g. by bringing about image-wise diffusion of a dye into an image-receiving layer from a silver halide emulsion layer or from an adjacent layer in water-permeable relationship with such emulsion layer, in dependence on the principle of dye solubility modification in function of the amount of photographic silver halide developed. The image-receiving layer used in a dye diffusion transfer imaging method usually comprises a mordant. A survey of dye diffusion transfer imaging processes has been given by Christian C. Van de Sande in Angew.Chem.-Int. Ed. Engl. 22 (1983) n° 3, 191-209. Nowadays use is mostly made of redox-controlled dye-releasing compounds that form image dyes on diffusion transfer into an image-receiving layer.

In any diffusion transfer imaging method, the image-receiving layer can be an integral part of the element incorporating the silver halide emulsion layer(s), the silver halide and image-receiving layers being carried by a common support. Alternatively, the silver halide emulsion layer(s) on the one hand and the image-receiving layer on the other hand can be layers of different elements that are brought together for the diffusion transfer operation.

The problem of image-dye fading under the influence of light has been the subject of extensive research for a long time, as is apparent from patent literature.

For instance, in DE-A 2,126,187 imidazolidine or oxazolidine compounds, which may comprise a polyalkylpiperidine group, have been proposed as anti-fading agents for dye images.

In DE-A 2,126,954 certain polyalkylpiperidines have been proposed as anti-fading agents for colour photographs.

In DE-A 2,647,452; 2,654,058; and 2,656,769 different hydroxybenzyl-substituted piperidine derivatives have been described.

In EP A 0,011,051 polyalkylpiperidine derivatives containing at least one phenol group have been described as anti-fading agents for colour photographs. These polyalkylpiperidine derivatives are polyalkylpiperidine esters of hydroxybenzylmalonic acids.

Other hydroxybenzyl-or phenol-substituted polyalkylpiperidine derivatives have been proposed for the same purpose in US A 4,452,884; 4,465,765; 4,465,757; 4,518,679; and 4,496,649; and in EP A 0,109,937 and 0,111,448.

In US A 4,517,283 a stabilizer mixture of a polyalkylpiperidine and a phenolic anti-oxidant has been proposed for anti-fading purposes.

Heterocyclic compounds, which may comprise a polyalkylpiperidine group, have been proposed for the same purpose in US A 4,518,688.

Hydroquinone-monoether or resorcinol-monoether-substituted polyalkylpiperidines have been proposed as anti-fading agents for colour photographs in EP A 0,114,029.

It is also known from DE A 3,246,292, for the purpose of avoiding the formation of stains upon development, to incorporate 4-acylamido-substituted polyalkylpiperidines in colour photographic light-sensitive silver halide elements comprising 2-equivalent magenta-forming 5-pyrazoline-type couplers.

Attempts are still being made to provide effective anti-fading agents.

It has now been found that the light-fastness of a dye image obtained according to a diffusion transfer imaging method can be improved and staining thereof prevented by using one or more piperidine derivatives as defined hereinafter, in conjunction with the dye.

According to the present invention there is provided a dye image-receiving element, which in an image-receiving layer or in a hydrophilic colloid layer in water-permeable relationship therewith comprises, as an anti-fading agent, at least one 2,2,6,6-tetramethyl-piperidine and/or at least one 2,2,6,6-tetramethyl-piperidinium salt and/or at least one polymer comprising recurring structural units, at least some of which are derived from a 2,2,6,6-tetramethyl-piperidine or from a 2,2,6,6-tetramethyl-piperidinium salt, characterised in that said 2,2,6,6-tetramethyl-piperidine and said 2,2,6,6-tetramethyl-piperidinium salt correspond to the general formulae I and II respectively :

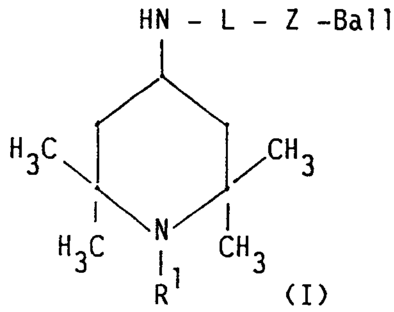
(I)

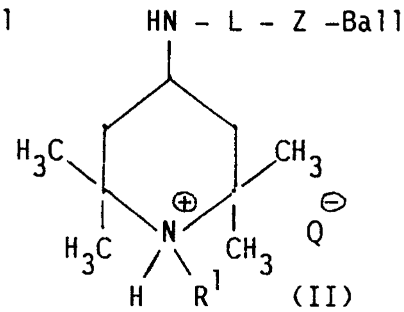
(II)

wherein:

L represents -CO-, -CO-CO-, or $-SO_2-$,

Z represents a single bond, -O-or $-N(R^2)-$, $R^2$ being hydrogen, a $C_1$-$C_{20}$alkyl group, a substituted $C_1$-$C_{20}$alkyl group, an aryl group, a substituted aryl group, an aralkyl group, a substituted aralkyl group, or a heterocyclic group,

Ball is a ballasting group of sufficient size to render said anti-fading agent non-diffusing in hydrophilic colloid media and containing no water-solubilizing group such as hydroxy, carboxy, or sulpho,

$R^1$ represents hydrogen, a $C_1$-$C_{20}$alkyl group e.g. methyl, a substituted $C_1$-$C_{20}$alkyl group, an aralkyl group e.g. benzyl, or a substituted aralkyl group,

$Q^-$ represents a mono-or polyatomic anion e.g. $Cl^-$, $HSO_4^-$ ,

and in that said recurring structural units derived from a 2,2,6,6-tetramethyl-piperidine or from a 2,2,6,6-tetramethyl-piperidinium salt are units derived from compounds of the above general formulae I and II wherein Ball represents one of the following moieties III or IV:

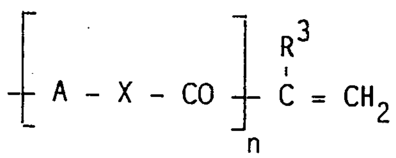
(III)

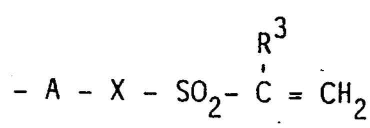
(IV)

wherein:

A represents a $C_1$-$C_6$alkylene group e.g. ethylene, a substituted $C_1$-$C_6$alkylene group, an arylene group, a substituted arylene group, an aralkylene group, or a substituted aralkylene group,

X represents a single bond, -O-, or $-N(R^4)-$, $R^4$ having one of the significances as defined for $R^2$ hereinbefore,

$R^3$ is hydrogen or a $C_1$-$C_4$alkyl group e.g. methyl, and

n is zero or 1.

In the general formulae I and II the ballasting group Ball can e.g. be a $C_5$-$C_{20}$alkyl group, a substituted $C_5$-$C_{20}$alkyl group, an alkaryl group, a substituted alkaryl group, an aralkyl group, or a substituted aralkyl group. Examples of preferred ballasting groups are n-pentadecyl, n-hexadecyl, n-octadecyl, and 4-n-hexadecyloxyphenyl.

The present invention also provides a dye image-carrying element comprising (a) layer(s) in which a magenta, cyan, and/or yellow dye has (have) been formed by diffusion transfer and comprising as an anti-fading agent for at least one of the dyes, a 2,2,6,6-tetramethyl-piperidine and/or a 2,2,6,6-tetramethyl-piperidinium salt corresponding to the above general formulae I and II respectively.

Although in this specification reference is more particularly exemplified by embodiments of the invention wherein use is made of an anti-fading agent comprising a single compound corresponding to one of the aforesaid formulae I and II, it is to be noted that the invention includes embodiments wherein the dye image-receiving or dye image-carrying element incorporates an anti-fading agent comprising two or more compounds selected from those covered by said general formulae.

The selected anti-fading agent can be incorporated in a dye image-receiving element prior to any processing thereof for forming the dye image. Alternatively said agent can be initially incorporated into a processing liquid with which the dye image-receiving or dye image-carrying element is wetted so that said agent becomes imbibed and becomes effectively present in the dye image-carrying element. The anti-fading agent can for example be incorporated into a treatment bath to be applied after the colour development. In particular, the agent can be incorporated into a stabilization bath employed in a final treatment of the dye image-carrying element.

In general, the anti-fading compounds according to formulae I and II above are sparingly soluble in water. However, they can usually be dissolved readily in a low boiling solvent such as methyl acetate, ethyl acetate, carbon tetrachloride, chloroform, methanol, ethanol, n-butanol, dioxan, acetone, benzene, dimethylformamide, or dimethyl sulfoxide; in a high boiling organic solvent such as tricresyl phosphate, di-n-butyl phthalate, or ethyl-N-diphenyl carbamate; or in a solvent mixture comprising two or more of the above-mentioned low boiling and/or high boiling organic solvents.

A solution of at least one said anti-fading compound in such solvent or solvent mixture can be added to a colloid solution such as e.g. an aqueous gelatin solution, then dispersed by means of a colloid mill or homogenizer, and finally incorporated e.g. into the coating composition for forming a mordanting layer of a dye image-receiving element.

In case the selected anti-fading agent is soluble in lower alcohol, alkali, or water, it can be added to a final treating bath.

Compounds according to formulae I and II can be incorporated into a layer-forming composition alone or in combination with other compounds, in the form of a dispersion either comprising no solvent or comprising high-boiling or low-boiling solvents or a mixture of such solvents. The anti-fading compounds can alternatively be incorporated together with a polymer in the form of a latex into a dye image-receiving element. They can be incorporated, alone or in combination with other compounds, into an image-receiving layer, which may comprise a mordanting agent. They can also be incorporated, alone or in combination with other compounds, into a layer, other than the image-receiving layer. This other layer is preferably one, which is closer to the silver halide emulsion layer(s) during diffusion transfer than the image-receiving layer. The compounds can be added in dispersed form to a composition forming e.g. an image-receiving layer, an intermediate layer, or a protective layer.

Dye image-receiving elements according to the invention can be processed in a known manner. Furthermore, they can be treated during or after the processing in a manner that further increases their stability e.g. by treatment in a stabilizing bath or by the application of a protective coating.

In certain cases, the anti-fading compounds according to the said formulae are also suitable for protecting dye images formed by selective bleaching of dyed photographic emulsion layers.

The amount of the anti-fading agent used can vary between wide limits. The amount is generally within the range from 1 to 3000 mg and is preferably from 500 to 2000 mg, per m2 of the layer in which it has been incorporated.

A dye image-receiving element may in addition to a said anti-fading agent also comprise one or more ultraviolet-absorbing compounds. Such compound(s) can be present in the same layer as the anti-fading agent or in an adjacent layer. The amount of ultraviolet-absorbing compound(s) can vary between wide limits. Generally, the amount will be in the range from 200 to 2000 mg, and it is preferably from 400 to 1000 mg per m2 of the layer. Examples of appropriate ultraviolet-absorbing compounds are those of the benzophenone, acrylonitrile, thiazolidone, benzotriazole, oxazole, thiazole, and imidazole type.

The dye images obtained with dye image-receiving elements according to the present invention by diffusion transfer have a very good stability against visible and ultraviolet radiation.

The anti-fading agents for use in accordance with the present invention are virtually colourless, so that no discolouration of the dye images results. They are readily compatible with the customary photographic additives present in the individual layers. By virtue of their good activity, they can be used in small amounts so that they do not precipitate or crystallize out, if they are incorporated as an organic solution into the aqueous binder emulsions used for the preparation of photographic layers.

Any material can be employed as image-receiving element in colour diffusion transfer photography, provided it presents the desired function of mordanting or otherwise fixing the dyes that diffuse or form therein. For details on image-receiving elements for use in dye diffusion systems in colour photography there can be referred to the above-mentioned survey of Christian C. Van de Sande in Angew.Chem. The selection of the particular material to be used is, of course, determined by the nature of the dye(s) to be mordanted. If acid dyes are to be mordanted, the image-receiving layer can be composed of or contain basic polymeric mordants such as polymers of amino-guanidine derivatives of vinyl methyl ketone such as described in US-A 2,882,156 of Louis M. Minsk, issued April 14, 1959, and basic polymeric mordants and

derivatives, e.g. poly-4-vinylpiperidine, the metho-p-toluene sulphonate of 2-vinylpiperidine and similar compounds described in US-A 2,484,430 of Robert H.Sprague and Leslie G.Brooker, issued October 11, 1949, and the compounds described in the published DE-A 2,200,063 filed January 11, 1971 by Agfa-Gevaert A.G. Suitable mordanting binders include, e.g., guanylhydrazone derivatives of acyl styrene polymers, as described, e.g., in published DE-A 2,009,498 filed February 28, 1970 by Agfa-Gevaert A.G. In general, however, other binders, e.g. gelatin, are added to the last-mentioned mordanting binders. Effective mordanting compositions are long-chain quaternary ammonium or phosphonium compounds or ternary sulphonium compounds, e.g. those described in US-A 3,271,147 of Walter M.Bush and 3,271,148 of Keith E.Whitmore, both issued September 6, 1966, and cetyltrimethyl-ammonium bromide. Certain metal salts and their hydroxides that form sparingly soluble compounds with the acid dyes can be used too. The dye mordants are dispersed in one of the usual hydrophilic binders for the image-receiving layer, e.g. in gelatin, polyvinylpyrrolidone, or partly or fully hydrolysed cellulose esters.

Customarily, good results are obtained when the image-receiving layer, which preferably is permeable to alkaline solution, is transparent and has a thickness of approximately 4 to 10 µm. Of course, the thickness can be modified depending upon the results aimed at.

The image-receiving layer can further contain brightening agents e.g. stilbenes, coumarins, triazines, oxazoles, or anti-oxidants such as the chromanols and alkyl-phenols.

Representatives of the anti-fading agents as defined above for use in accordance with the present invention are listed in the following Table 1.

TABLE 1

Compound

N°

1 2,2,6,6-tetramethyl-4-palmitamido-piperidine
2 1,2,2,6,6-pentamethyl-4-palmitamido-piperidine
3 2,2,6,6-tetramethyl-4-n-octadecylureido-piperidine
4 1,2,2,6,6-pentamethyl-4-n-octadecylureido-piperidine
5 2,2,6,6-tetramethyl-4-hexadecyloxycarbonylamino-piperidine
6 1-benzyl-2,2,6,6-tetramethyl-4-hexadecyloxycarbonylamino-piperidine
7 2,2,6,6-tetramethyl-4-hexadecanoylcarbonylamino-piperidine
8 2,2,6,6-tetramethyl-4-hexadecylsulphonamido-piperidine
9 2,2,6,6-tetramethyl-4-(p-hexadecyloxyphenyl)-sulphonamido-piperidine
10 1-benzyl-2,2,6,6-tetramethyl-4-(p-hexadecyloxyphenyl)-sulphonamido-piperidine
11 1-benzyl-2,2,6,6-tetramethyl-4-hexadecylsulphonamido-piperidine
12 copolymer corresponding to the formula:

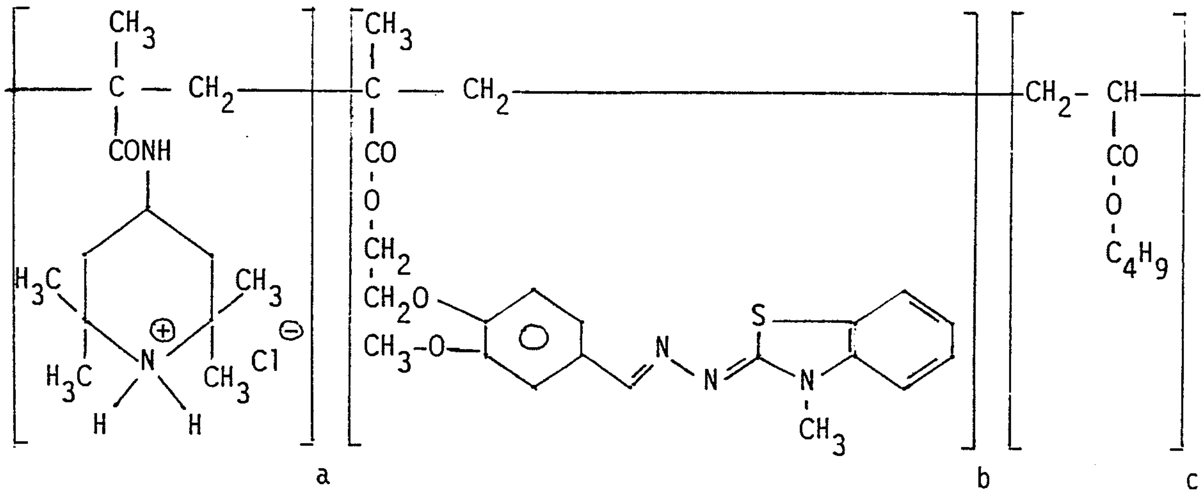

wherein a = 33 ; b = 54 ; and c = 13 % by weight

13 copolymer corresponding to the formula of compound 12, but wherein a = 22.5 ; b = 0 ; and c = 77.5 % by weight

14 copolymer corresponding to the formula:

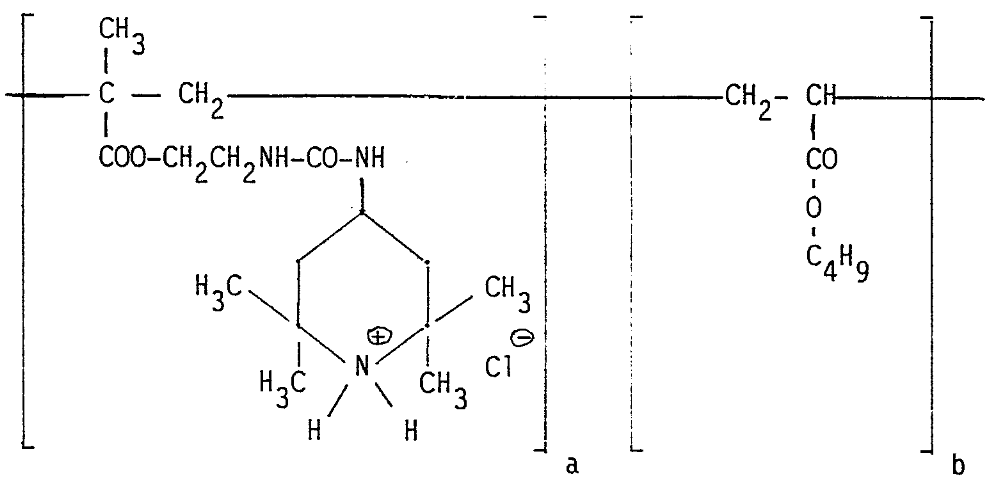

wherein a = 40 ; b = 60 % by weight

The following preparations illustrate the synthesis of compounds for use as anti-fading agents in accordance with the invention.

PREPARATION 1: Compound 1

A solution of 1.74 g (0.00585 mol) of palmitoyl chloride in 10 ml of methylene chloride was added dropwise to a solution of 1 ml (0.0056 mol) of 4-amino-2,2,6,6-tetramethyl-piperidine in 10 ml of methylene chloride. The mixture was stirred for 1.5 h at 25°C. The methylene chloride was removed by evaporation. A volume of 40 ml of diethyl ether was added. The resulting mixture was heated to form a grainy precipitate, which was filtered and dried.
Yield: 2.2 g (88%) of the waxy Compound 1. Melting point: 218°C.

PREPARATION 2: Compound 3

A solution of 4 ml (0.011 mol) of octadecyl isocyanate and 1.76 ml (0.011 mol) of 4-amino-2,2,6,6-tetramethyl-piperidine in 10 ml of toluene was stirred for 1 h at room temperature. The solvent was removed by evaporation under reduced pressure. A clear oil crystallizing under reduced pressure was obtained.
Yield: 4.4 g (98%) of Compound 3. Melting point: 70°C.

PREPARATION 3: Compound 14

a) synthesis of 4-(2-methacryloyloxy-ethyl)-ureido-2,2,6,6-tetramethyl-piperidinium chloride

An amount of 24.8 g (0.16 mol) of isocyanato-ethyl methacrylate was added dropwise in 1 h to a solution of 25 g (0.16 mol) of 4-amino-2,2,6,6-tetramethyl-piperidine in 150 ml of dichloromethane. The mixture was cooled to keep the temperature at 20°C and was stirred for 1 h. Hydrogen chloride gas was introduced for 30 min. A volume of 250 ml of diethyl ether was added. The resulting precipitate was filtered with suction.
Yield: 54 g (97%) of 4-(2-methacryloyloxy-ethyl)-ureido-2,2,6,6-tetra-methyl-piperidinium chloride.

b) synthesis of Compound 14

A volume of 200 ml of water was heated to 90°C under nitrogen athmosphere. A volume of 6 ml of a 1% initiator solution of the sodium salt of 4,4-azo-bis-4-cyanovaleric acid in water was added thereto. After 5 min a suspension of 20 g of 4-(2-methacryloyloxy-ethyl)-ureido-2,2,6,6-tetramethyl-piperidinium chloride and 2.5 g of n-hexadecyl-pyridinium chloride in 100 ml of water was added dropwise in 30 min and simultaneously a continuous flow of 30 g of butyl acrylate and 19 ml of the same 1% initiator as described above was introduced at a polymerization temperature of 94-97°C. After 1 h the remaining monomer was removed by evaporation. A volume of 260 ml of fine grain latex having a content of solids of 17.1 % was obtained.

PREPARATION 4: Compound 13

a) synthesis of 4-methacrylamido-2,2,6,6-tetramethyl-piperidinium chloride

A solution of 6.7 ml (0.068 mol) of methacryloyl chloride in 40 ml of dichloromethane was added in 15 min to a stirred solution of 10.6 g (0.068 mol) of 4-amino-2,2,6,6-tetramethyl-piperidine in dichloromethane. After 3 h of stirring the resulting precipitate was collected and rinsed with 50 ml of dichloromethane.
Yield: 13 g (78%) of 4-methacrylamido-2,2,6,6-tetramethyl-piperidinium chloride.

b) synthesis of Compound 13

The synthesis of Compound 13 was analogous to that described in Preparation 3 (b) but, of course, with 4-methacrylamido-2,2,6,6-tetra-methyl-piperidinium chloride instead of 4-(2-methacryloyloxy-ethyl)-ureido-2,2,6,6-tetramethyl-piperidinium chloride .

Other compounds for use as anti-fading agents in accordance with the invention and corresponding to the above general formulae can be prepared analogously or by techniques known in the art starting with the appropriate chemicals.

The following examples illustrate the present invention.

EXAMPLE 1

Preparation of photosensitive elements

A strip of subbed polyethylene terephthalate support having a thickness of 0.1 mm was coated with the following layers in the given order:

1) silver halide emulsion layer containing:

1.8 g gelatin

0.5 g silver chloride expressed as silver nitrate

0.09 g electron donor compound :

2,5-bis(1',1',3',3'-tetramethyl-butyl)-hydroquinone

0.333 g yellow dye-releasing compound D1 according to the following formula:

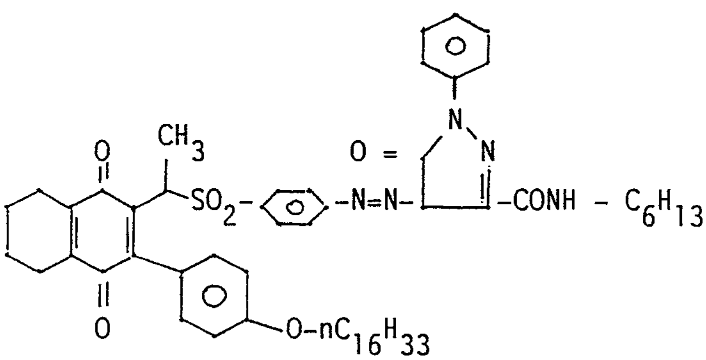

(D1)

2) protective layer containing:

6 g gelatin

0.12 g 1-phenyl-4-methyl-pyrazolidin-3-one

0.06 g citric acid up to a pH of 4.5 in both layers

A number of identical strips of photosensitive element were made, except that each of them contained instead of the above-mentioned dye-releasing compound D1 an equal molar concentration of the dye-releasing compounds D2 or D3 corresponding to the following formulae:

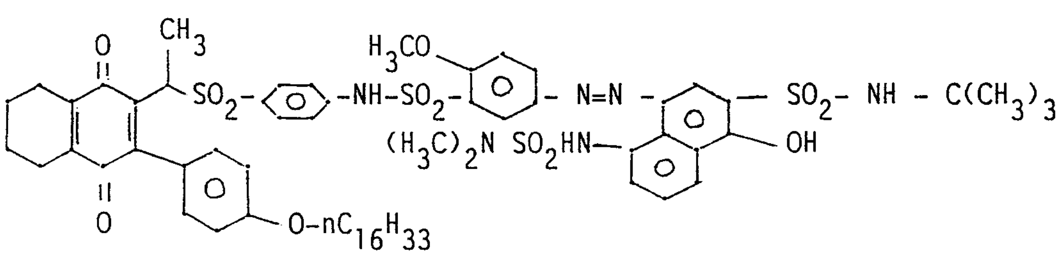

(D2)

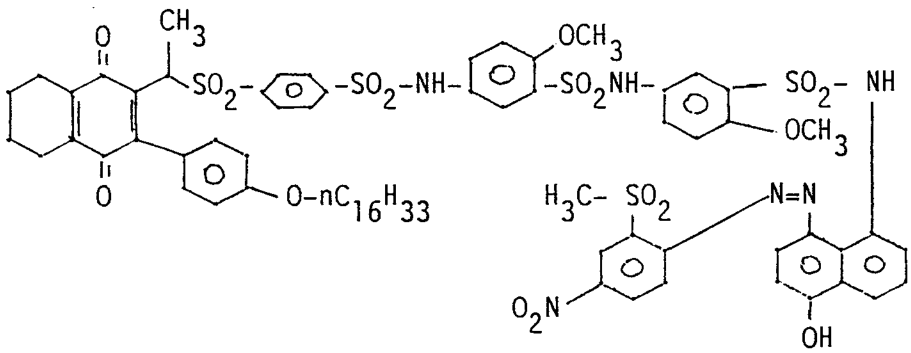

(D3)

Preparation of dye image-receiving elements

Strips of corona-treated polyethylene-coated paper support were coated with the following image-receiving layer (first layer) and an antistress layer, the first layer of the different strips comprising its ingredients in the different concentrations used in the Tests 1, 2, and 3 listed in Table 2:

1) first layer comprising gelatin, polymeric mordant prepared from 4,4'-diphenylmethane diisocyanate and N-ethyldiethanolamine quaternized with epichlorohydrin as described in Example 1 of published DE-A 2,631,521, and an anti-fading agent as identified in Table 2,

2) antistress layer comprising gelatin.

A dye image-receiving element containing no anti-fading agent at all was used as comparison.

The photosensitive elements were exposed image-wise and together with the image-receiving elements as described above fed through a COPYPROOF (trade mark of Agfa-Gevaert N.V. Belgium) CP 42 diffusion transfer processing apparatus containing in its tray an aqueous alkaline processing liquid comprising per litre:

25 g sodium hydroxide
25 g sodium orthophosphate
80 g cyclohexane dimethanol
2 g sodium bromide
2 g sodium thiosulphate, and
a sufficient volume of water to make 1 litre.

After the diffusion transfer took place, the photosensitive elements were stripped from the dye image-receiving elements. The stability to light of the dyes transferred to the dye image-receiving elements and mordanted therein was tested with a XENOTEST (trade mark) type 50 apparatus of Hanau Quartzlampen GmbH, Hanau, W.Germany. The different strips of dye image-receiving element (receptor) containing the mordanted dyes were exposed for 8 h to white light and ultra-violet radiation. The % loss in maximum density of mordanted dye is given in Table 2.

TABLE 2

| Composition of receptor | | Amounts in g/m2 | | | |
|---|---|---|---|---|---|
| | | Test n°1 | n°2 | n°3 | Comparison |
| 1st layer | gelatin | 3.6 | 5 | 7.6 | 4 |
| | polymeric mordant | 2.66 | 2.66 | 2.66 | 2.66 |
| | compound 1 | 1 | - | - | - |
| | compound 3 | - | 0.66 | 1 | - |
| antistress | gelatin | 0.66 | 0.66 | 0.66 | 0.66 |
| Mordanted dye deriving from dye-releasing compound | | % loss in maximum density | | | |
| | D1 | -28 | -22 | -11 | -34 |
| | D2 | -13 | -12 | - 7 | -17 |
| | D3 | -32 | -32 | -28 | -55 |

The results set forth in Table 2 clearly show that when the anti-fading agents as defined above are present in dye images obtained by diffusion transfer, the prevention from fading of these dyes is enhanced considerably.

## EXAMPLE 2

Strips of photosensitive elements comprising the above-mentioned dye-releasing compound D3 were prepared as described in Example 1.

Strips of dye image-receiving elements were made also as described in Example 1, the first layer and the antistress layer of the different strips comprising their ingredients in the different concentrations used in the Tests 4 to 8 listed in Table 3. A dye image-receiving element containing no anti-fading agent at all was used as comparison.

The photosensitive elements were exposed image-wise and together with the dye image-receiving elements fed through a processing apparatus as described in Example 1 and containing the processing liquid described therein.

After diffusion transfer the photosensitive elements were stripped from the dye image-receiving elements. The stability to light of the dyes transferred to the dye image-receiving elements and mordanted therein was tested in the same way as described in Example 1. The % loss in maximum density of mordanted dye is given in Table 3.

TABLE 3

| Composition of receptor | Amounts in g/m2 | | | | | |
|---|---|---|---|---|---|---|
| | Test n°4 | n°5 | n°6 | n°7 | n°8 | Comparison |
| 1st layer: | | | | | | |
| gelatin | 3.37 | 3.37 | 3.37 | 3.37 | 3.37 | 3.37 |
| polymeric mordant | 2.4 | 2.4 | 2.4 | 2.4 | 2.4 | 2.4 |
| compound 12 | - | 2 | - | - | 2 | - |
| compound 13 | 2 | - | - | - | - | - |
| antistress layer: | | | | | | |
| gelatin | 1 | 1 | 1 | 1 | 1 | 1 |
| compound 12 | - | - | - | 1 | - | - |
| compound 13 | - | - | 1 | | 1 | - |
| Mordanted dye deriving from dye-releasing compound | % loss in maximum density | | | | | |
| D3 | -33 | -39 | -40 | -41 | -38 | -49 |

An amount of 1 g of compound 12 corresponds with 1.26 millimole; 1 g of compound 13 corresponds with 1.15 millimole.

The results shown in Table 3 demonstrate that the prevention from fading of the image dyes is enhanced considerably by the presence of the anti-fading agents of the present invention.

**Claims**

1. Dye image-receiving element, which in an image-receiving layer or in a hydrophilic colloid layer in water-permeable relationship therewith comprises, as an anti-fading agent, at least one 2,2,6,6-tetramethyl-piperidine and/or at least one 2,2,6,6-tetramethyl-piperidinium salt and/or at least one polymer comprising recurring structural units, at least some of which are derived from a 2,2,6,6-tetramethyl-piperidine or from a 2,2,6,6-tetramethyl-piperidinium salt, characterised in that said 2,2,6,6-tetramethyl-piperidine and said 2,2,6,6-tetramethyl-piperidinium salt correspond to the general formulae I and II respectively:

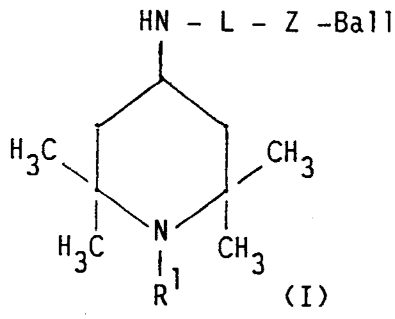
(I)

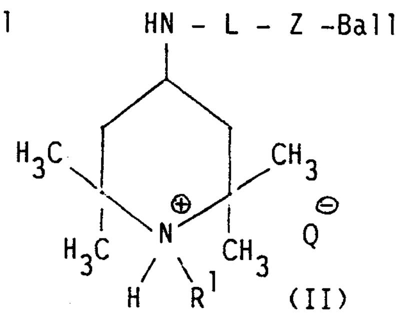
(II)

wherein:
L represents -CO-, -CO-CO-, or $-SO_2-$,
Z represents a single bond, -O-or $-N(R^2)-$, $R^2$ being hydrogen, a $C_1$-$C_{20}$alkyl group, a substituted $C_1$-$C_{20}$alkyl group, an aryl group, a substituted aryl group, an aralkyl group, a substituted aralkyl group, or a heterocyclic group,
Ball is a ballasting group of sufficient size to render said anti-fading agent non-diffusing in hydrophilic colloid media and containing no water-solubilizing group,
$R^1$ represents hydrogen, a $C_1$-$C_{20}$alkyl group, a substituted $C_1$-$C_{20}$alkyl group, an aralkyl group, or a substituted aralkyl group,
$Q^-$ represents a mono-or polyatomic anion,
and in that said recurring structural units derived from a 2,2,6,6-tetramethyl-piperidine or from a 2,2,6,6-tetramethyl-piperidinium salt are units derived from compounds of the above general formulae I and II wherein Ball represents one of the following moieties III or IV:

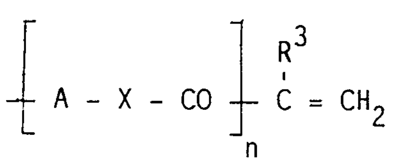
(III)

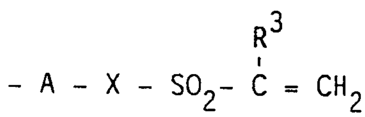
(IV)

wherein:
A represents a $C_1$-$C_6$alkylene group, a substituted $C_1$-$C_6$alkylene group, an arylene group, a substituted arylene group, an aralkylene group, or a substituted aralkylene group,
X represents a single bond, -O-, or $-N(R^4)-$, $R^4$ having one of the significances as defined for $R^2$ hereinbefore,
$R^3$ is hydrogen or a $C_1$-$C_4$alkyl group, and
n is zero or 1.

2. A dye image-receiving element according to claim 1, characterised in that it contains from 1 to 3000 mg of said anti-fading agent per m2 of the layer, in which it has been incorporated.

3. A dye image-receiving element according to claim 2, characterised in that it contains from 500 to 2000 mg of said anti-fading agent per m2 of the layer, in which it has been incorporated.

4. Dye image-carrying element comprising (a) layer(s) in which a magenta, cyan, and/or yellow dye image has (have) been formed by diffusion transfer and comprising as an anti-fading agent for the dye(s), a 2,2,6,6-tetramethyl-piperidine and/or a 2,2,6,6-tetramethyl-piperidinium salt and/or a polymer comprising

recurring structural units, at least some of which are derived from a 2,2,6,6-tetramethyl-piperidine or from a 2,2,6,6-tetramethyl-piperidinium salt, characterised in that said 2,2,6,6-tetramethyl-piperidine and said 2,2,6,6-tetramethyl-piperidinium salt and said polymer are as defined in claim 1.

European Patent Office

# EUROPEAN SEARCH REPORT

Application number

EP 86 20 2013

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | DE-A-3 246 292 (FUJI PHOTO FILM)<br>* Claims 1,20; page 16, line 22 - page 17, line 14; page 43, compound II-10 * | 1-4 | G 03 C 7/26<br>G 03 C 5/54 //<br>C 07 D 211/58<br>C 08 F 220/38 |
| Y | EP-A-0 093 924 (AGFA-GEVAERT)<br>* Page 9, formula C4; page 11, lines 10-15; page 17, line 4 - page 18, line 17; page 19, last paragraph - page 20, line 5; claims 1,7 * | 1-4 | |
| Y | FR-A-2 232 776 (EASTMAN KODAK)<br>* Pages 5-11, table I; page 13, lines 5-11; claims 1,6 * | 1-4 | |
| Y | DE-A-2 517 223 (FUJI PHOTO FILM)<br>* Page 10, line 8 - page 12, line 9; page 13, lines 1-7 * | 1-4 | **TECHNICAL FIELDS SEARCHED (Int. Cl.4)**<br>G 03 C 7<br>G 03 C 5<br>G 03 C 1 |
| D,A | EP-A-0 104 146 (CIBA-GEIGY) | | |
| A | DE-A-2 738 903 (FUJI PHOTO FILM) | | |

The present search report has been drawn up for all claims

| Place of search | Date of completion of the search | Examiner |
|---|---|---|
| THE HAGUE | 03-03-1987 | PHILOSOPH L.P. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document